(19)

Europäisches
Patentamt
European
Patent Office
Office européen
des brevets

(11)     EP 4 386 352 A1

(12)     **EUROPEAN PATENT APPLICATION**

(43) Date of publication:
**19.06.2024   Bulletin 2024/25**

(21) Application number: **22213113.8**

(22) Date of filing: **13.12.2022**

(51) International Patent Classification (IPC):
**G01N 1/31** *(2006.01)*        **C12N 1/06** *(2006.01)*
**C12N 15/10** *(2006.01)*       *G01N 1/08* *(2006.01)*
*G01N 1/34* *(2006.01)*          *G01N 35/10* *(2006.01)*

(52) Cooperative Patent Classification (CPC):
**G01N 1/312; C12N 1/06; C12N 1/066;** G01N 1/08;
G01N 1/34; G01N 2035/103; G01N 2035/1048

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Tecan Trading AG
8708 Männedorf (CH)**

(72) Inventors:
• **STRASSEN, Simon
Männedorf (CH)**
• **VILAJ, Volfgang
Uetikon am See (CH)**

(74) Representative: **DeltaPatents B.V.
Fellenoord 370
5611 ZL Eindhoven (NL)**

(54)     **METHOD FOR PLANNING TREATMENT OF AN AREA OF INTEREST ON A TISSUE SECTION AND FOR FACILITATING TISSUE TREATMENT**

(57)     Some embodiments are directed to planning treatment of an area of interest on a tissue section with a motorized treatment tip. The planning may include computing for each pixel in the area of interest a distance to the nearest pixel in the tissue section outside the area of interest or in the background, and selecting a position for a treatment area from the result of the distance transform.

Fig. 1

EP 4 386 352 A1

## Description

## TECHNICAL FIELD

the presently disclosed subject matter relates to a planning treatment of an area of interest on a tissue section, a system for planning treatment of an area of interest on a tissue section method for treating an area of a tissue section, and a computer readable medium.

## BACKGROUND

The international patent application WO2020131070, with title "Method of treating a sample", and included herein by reference, discloses a method of treating an isolated area of a sample with a liquid. A known method disclosed therein comprises the steps of:

- generating an isolated area of a sample by means of a first fluid reservoir, the first fluid reservoir enclosing a distal end of a second fluid reservoir, and the isolated area of the sample being sealed towards the remaining area of the sample,
- bringing the isolated area of the sample into fluid connection with the second fluid reservoir,
- dispensing a fluid from the second fluid reservoir into the first fluid reservoir, thereby generating a fluid flow on the sample at the isolated area in a first direction, and
- aspirating the fluid from the first fluid reservoir into the second fluid reservoir, thereby generating a fluid flow on the sample at the isolated area in a second direction.

For example, figure 9 of the above patent application shows a microscope image of a formalin fixed paraffin embedded (FFPE) section of a tissue. The corresponding description gives an example of treatment of the slide comprising the tissue section.

When working with the known method of treating a sample, users need to indicate where to place the fluid reservoirs, e.g., detachment chambers or extraction pods, on the tissue sample. For example, a user, such as a pathologist, marks one or more areas as areas of interest. An operator of the known method then determines where treatment will take place, e.g., where extraction pods need to be placed. Such placement is not easy, using too many pods will increase extracting time, using too few will decrease the amount of detached tissue. Furthermore, detachment areas may partially overlap, or may, to some extent, extend outside the area of interest. The latter will cause some tissue that is not desired to be detached, but some percentage of this may be allowed. For a human operator making a good choice amongst all of these possibilities is hard. To make matters worse, typically detachment chambers come in different sizes. Large chambers having the advantage that a large part of tissue can be detached in one go, but smaller chambers have the advantage that they can be placed more precisely, e.g., avoiding overlap amongst detachment areas or avoiding extending outside the area of interest.

## SUMMARY

There is a need for algorithms that generates a treatment plan for a given area of interest. Preferably, the algorithm takes into account a treatment device that supports different shapes, e.g., different sizes. Preferably, the algorithm may be tuned by a user through parameters to support different use cases, e.g. different downstream processes.

Generating treatment plans is vulnerable to the so-called combinatorial explosion. Consider the number of placement for one treatment area to be $O(n)$, wherein n is related to the number of possible positions for placement, say the number of pixels in an image of the tissue, area of interest and background, say, of a tissue slide. Should one consider the placement of multiple treatment areas, the work then rises to $O(n^k)$, where $k$ is the number of considered areas. This quickly becomes unpracticable.

A clever way to consider placements one at a time, while still obtaining placement that allow later treatment areas to be placed well, is to apply a distance transform to the distance to the nearest pixel in the tissue section outside the area of interest or in the background, e.g., to the edge of the area of interest. The distance transform has a preferred value for a radius of the geometric shape and has less preferred values for values higher and/or lower than the radius. This has the effect that optimizing for the distance transformed values causes the treatment areas to be preferentially placed close the edge of the area of interest.

Using the distance transform for selecting treatment areas has the effect of placing them at or near the edge of the area of interest. This then naturally allows scope for the placement of future treatment areas, without having to explicitly take them into account.

The distance transformation is correlated to the approximate gain, that treatment, say detachment, at that location will bring, but in an embodiment, expected gain is explicitly taken into account. This increases the gain of the treatment plans. For example, in an embodiment the expected detachment gain from a selected detachment area is computed, and taken into account for selecting a position for a detachment area.

## BRIEF DESCRIPTION OF DRAWINGS

Further details, aspects, and embodiments will be described, by way of example only, with reference to the drawings. Elements in the figures are illustrated for simplicity and clarity and have not necessarily been drawn to scale. In the figures, elements which correspond to elements already described may have the same reference numerals. In the drawings,

Figure 1 schematically shows an example of an embodiment of a tissue treatment device,

Figure 2a schematically shows an example of an embodiment of a tissue treatment system,

Figure 2b schematically shows an example of an embodiment of a tissue treatment system,

Figure 3a schematically shows an example of an embodiment of a treatment planning system,

Figure 3b schematically shows an example of an embodiment of a treatment planning method,

Figure 4a schematically shows an example of an embodiment of treatment planning,

Figure 4b schematically shows an example of an embodiment of treatment planning,

Figure 5a schematically shows an example of an embodiment of an image showing tissue and markings,

Figure 5b schematically shows an example of an embodiment of an image showing a tissue mask,

Figure 5c schematically shows an example of an embodiment of an image showing partial contours,

Figure 5d schematically shows an example of an embodiment of an image showing a completed contour,

Figure 5e schematically shows an example of an embodiment of an image showing an isolated area of interest,

Figure 5f schematically shows an example of an embodiment of an image showing a treatment plan superimposed on an image of tissue

Figure 6a schematically shows an example of an embodiment of an image showing tissue and markings,

Figure 6b-6d schematically shows an example of an embodiment of an image showing a treatment plan superimposed on an image of tissue,

Figure 7 schematically shows an example of an embodiment of a treatment planning method,

Figure 8a schematically shows a computer readable medium having a writable part comprising a computer program according to an embodiment,

Figure 8b schematically shows a representation of a processor system according to an embodiment.

## Reference signs list

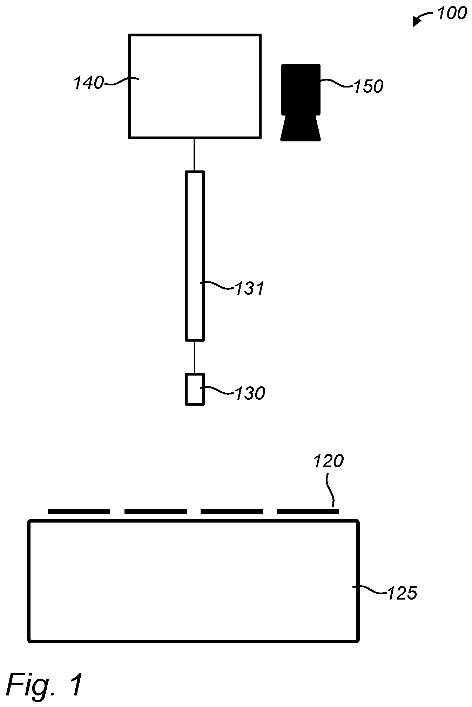

The following list of references and abbreviations corresponds to figures 1-6d, 8a, 8b, and is provided for facilitating the interpretation of the drawings and shall not be construed as limiting the claims.

| | |
|---|---|
| 100 | a tissue treatment device |
| 120 | a slide |
| 125 | a slide support |
| 130 | treatment tip |
| 131 | a treatment arm |
| 140 | a tissue treatment unit |
| 150 | a camera |
| 161 | an image with tissue section and markings, |

| | |
|---|---|
| 162 | a mask contour image |
| 163 | a mask tissue image |
| 164 | an isolated area of interest mask |
| 165 | a treatment plan superimposed on image 161 |
| 200, 201 | a treatment system |
| 210 | a treatment planning device |
| 230, 270 | a processor system |
| 240, 280 | a storage |
| 250, 290 | a communication interface |
| 260 | a tissue treatment device |
| 265 | a treatment apparatus |
| 266 | a camera |
| 300 | a treatment planning system |
| 310 | image preprocessing part |
| 311 | a contour image |
| 312 | a tissue image |
| 313 | a tissue and contours image |
| 320 | a treatment planning unit |
| 322 | a distance determination and distance transform unit |
| 324 | a gain and constraints determination unit |
| 340 | a treatment unit |
| 350 | an image filtering unit |
| 401-409 | an image |
| 410 | tissue |
| 420 | a surrounded area |
| 421 | a contour |
| 422 | an area of interest mask |
| 423 | an area of interest |
| 424 | a first detachment area |
| 425 | a removed detachment area |
| 426 | a second detachment area |
| 427 | a treatment plan |
| 620 | area of interest |
| 610 | tissue |

| | |
|---|---|
| 1000, 1001 | a computer readable medium |
| 1010 | a writable part |
| 1020 | a computer program |
| 1110 | integrated circuit(s) |
| 1120 | a processing unit |
| 1122 | a memory |
| 1124 | a dedicated integrated circuit |
| 1126 | a communication element |
| 1130 | an interconnect |
| 1140 | a processor system |

## DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the subject matter that is presently disclosed is not limited to the embodiments only, but also includes every other combination of features described herein or recited in mutually different dependent claims.

**Figure 1** schematically shows an example of an embodiment of a tissue treatment device 100. Tissue treatment device 100 is an example of a tissue treatment device that may be used in or with an embodiment, e.g., an embodiment of a treatment planning method or treatment planning device. Tissue treatment device 100 may have treatment planning functionality, e.g., comprise a treatment planning device, or may be combined with a treatment planning device.

Shown in figure 1 is a slide 120. Slide 120 is arranged for a tissue section, e.g., tissue slice, to be applied to a slide surface. Parts of the tissue section are to be treated, in particular, tissue may be detached from the location. A particular important example of tissue treatment is tissue detachment. Tissue detachment may be done by lysing. For example, a lysing fluid may be applied at a location by a lysing tip, e.g., a pipetting tip, the lysed tissue may then be aspirated back. The lysed tissue can then be used for further processing, e.g., to extract biological molecules, e.g., proteins, DNA molecules, etc. For example, the tissue may be a biopsy tissue. Slide 120 is shown on top of a slide support 125. Tissue detachment may be done by scraping or by laser instead; for example, the treatment tip may comprise a scraping tip. With the lysing a localized detachment of tissue is obtained.

Treatment may include chemical treatment, e.g., chemical detachment, e.g., lysing, local staining, or other local chemical treatment. Treatment may include mechanical treatment, e.g., mechanical detachment, e.g., scraping. Treatment may include thermal treatment, UV treatment, etc.

Tissue treatment device 100 comprises a treatment arm 131 with a treatment tip 130 at an end of the treatment arm. The slide surface of slide 120 is facing the treatment tip 130. Treatment tip 130 is movable and can be configured for treatment tip 130 to move to a particular defined location on the tissue section. Typically, treatment arm 131 is motorized and arranged to be controlled by a program. The program may instruct the treatment arm 131 and tip 130 for tissue treatment at one or more locations on the tissue section. For example, treatment arm 131 may be part of a robotic arm arranged to move tip 130 to a desired location on the tissue section. For example, the treatment tip may comprise a pipetting tip, e.g., for chemical detachment of tissue. For example, a pipetting tip may be arranged for application of a fluid and/or aspiration of a fluid, e.g., for lysing the tissue at the location. A pipetting tip may be configured to enable the controlled exposure of chemicals to the tissue at the defined location. The tip may also allow dynamic fluid forces at the location to further promote tissue treatment of the tissue section at the location. For example, shear forces may be applied to the tissue through the fluid. For example, the treatment tip may be a scraper, e.g., for mechanical and localized detachment of tissue.

In an embodiment, treatment tip 130 is arranged to extract biomolecules from the tissue material, such as one or more of nucleic acids, proteins, lipids, and hormones. For example, a pipetting tip may be configured to lyse the tissue material, and to aspirate the lysate. For example, a scraping tip may be configured to scrape the tissue material. From the lysate or scraped tissue, biomolecules can be extracted, in particular, DNA molecules, more in particular double-stranded DNA molecules (dsDNA).

The location on the tissue section, e.g., a part or area or region of the tissue section, comprises the material that is to be detached, e.g., to be lysed. The location is also referred to as the area of interest (AoI). The size of the location may be determined by the size of the treatment tip 130. Often a circular shape is taken for the treatment tip 130, and for the location, but this is not necessary. For example, the location may comprise a circular area defined by the corresponding inner diameter of a tissue treatment chamber. Other shapes, say triangular, or the like is possible, and may even be advantageous if, say multiple locations are to be combined to maximize the amount of tissue detached from the tissue section. For example, the location may comprise an area whose shape is defined by the corresponding shape of a tissue treatment chamber. The location may comprise an area defined by the corresponding dimension of a scraping pipetting tip. The size of the location may be defined by a spot size or beam diameter of a laser beam used for treatment, e.g., the half-power beam width.

A tissue treatment unit 140 may be configured to move treatment tip 131 to the location on the tissue section, and to treat the tissue as appropriate, e.g., supply, and aspirate fluids to and from treatment tip 131, and/or scrape the tissue, and so on.

Further information on and examples of pipetting tips can be found, e.g., in international patent publications WO2020131072 and WO2020132394, both of which are included herein by reference.

By way of example, a small area of interest may have a surface of about 2 mm$^2$, with a dispense aperture having for example a radius of about 0.79 mm. A small area of interest may for example have a diameter of about 1 mm. A medium-sized area of interest may for example have a surface of about 10 mm2, with the dispense aperture of the pipette tip extension having for example a radius of about 1.8 mm. A medium-sized area of interest may for example have a diameter of about 4 mm.

In an exemplary embodiment, the dispense aperture at a distal end of the pipette tip extension may have a circular shape or a circular cross-section, respectively, viewed orthogonally to the medial axis of the pipette tip extension. The cross-section of the dispense aperture may however depart from a circular shape, for example may be oval, triangular or may have another form, for example a polygonal form. The shape and/or the size of the dispense aperture may for example be adapted to a specific application or use of the pipette tip extension, for example to a specific area of interest of a tissue section which shall be addressed. Exemplarily, a particularly suitable size of a dispense aperture with an essentially circular shape may have a diameter of 1.65 mm. Suitable diameters may be in the range of 0.2 mm to 8 mm, in particular in the range of 1 to 2 mm. In an embodiment, the largest sized dispensing aperture has a radius 7.5 mm.

In an exemplary embodiment, the lysing area, e.g., the area of interest, may have a surface area size of 0.01 mm$^2$ or more, preferably, 0.1 mm$^2$ or more, more preferably 1 mm$^2$ or more. For example, the surface area size may be in a range from 0.01 mm$^2$ to 200 mm$^2$, although values above or below this range are possible. For example, the surface area size may be 8.5 mm$^2$, 12.5 mm$^2$, or 75 mm$^2$ or more, or less.

Further information on and examples of mechanical detachment tips, e.g., scraping can be found, e.g., in international patent publications WO2020254250A1, which is included herein by reference. Said publication discloses an apparatus comprising a dissection tool for mechanical detachment of biological material from a tissue sample disposed on a slide. A gouging head is configured to engage with the slide, relative movement between the platform and the dissection tool causes a front face of the gouging head to gouge a track though the tissue sample.

Returning to figure 1, by moving the pipetting tip to a location of interest and detaching the tissue at that location, some part of the tissue is obtained in unit 140 where it can be further processed. Detachment of tissue in this manner, especially automated or partially automated, is advantageous, as it is quick and reliable.

For example, tissue may be detached by one or more lysing iterations. In a lysing iteration, lysing fluid may be provided to the lysis chamber at the end of the pipetting tip, and after some time, aspirated back together with lysed material. The time the lysing fluid is in the chamber, as well as other factors, have an impact on the amount of material that is detached from the tissue slide.

A camera 150 may be included in tissue treatment device 100 to take images of the tissue section in various stages of tissue detachment. For example, an embodiment may comprise taking a first image, moving the treatment tip into position to detach tissue at the defined location. An image may also be taken before the first lysing, e.g., from a possibly stained tissue slice, possibly using different lighting, e.g., bright field. For example, camera 150 may be used to take a first image of a tissue slice, e.g., using bright field lighting, of a first tissue slice. Locations may be identified on the first tissue slice, e.g., by a user, for example, to identify at-risk tissue in the tissue slice, e.g., to identify cancer cells in the tissue slice. The markings of the user define an area of interest where the tissue is to be treated, e.g., detached, e.g., lysed or scaped.

The slide 120 containing the tissue slice may then be removed, e.g., motorized, and a second slide with a second slice may be moved under camera 150 and treatment tip 130. Typically, the second tissue slice is used for treatment. This tissue is typically not stained. Camera 130 may take a second image of the second tissue slice and use it to guide the treatment tip to the area of interest defined by the markings on the first slice. Using two or more tissue slices, which may be on two or more slides, allows marking and planning to use a stained tissue, while detachment may be done on an unstained tissue. However, this is not necessary, detachment could be done on the first tissue section.

Moving the treatment tip to and from the defined location may be done with a movable, e.g., motorized arm. For example, a robotic arm may be used. In an embodiment camera 150 may be used to guide arm towards the defined location, although this is not necessary. Slide 120 may comprise one or more fiducials to aid in locating the defined location in the camera image. Camera 150 and/or said fiducials may be used by guiding software configured to guide treatment arm 131 to the defined location.

In an embodiment, the treatment tip is moved parallel to tissue slide 120, creating an optical path from camera 150 to the tissue slice. In figure 1, the camera is shown above the tissue section, but this is not necessary. For example, the optical path may comprise angles, e.g., by including one or more mirrors in the optical path. This allows the camera to be located at a different position, away from the treatment unit.

In an embodiment, the treatment tip is moved orthogonal to tissue slide 120, creating an optical path from camera 150 to the defined location. For example, camera 150 may be attached to pipetting tip 130 or arm 131. By moving orthogonally away from tissue slide 120, an optical path is created for camera 150 to take an image of the tissue slice.

Combinations of parallel and/or orthogonal movement are possible, with or without using optical elements such as mirrors, optical fibers, and the like. The camera may be a conventional camera or a fiber optic camera.

The treatment tip may be provided with an extraction pod or detachment chamber for placement on the tissue. The pod may be selected from multiple pod sizes and/or pod shapes. Which pod to use and the location to place it may be described in a treatment plan. For example, a treatment plan may be a sequence of a pod identifier, and coordinates of a location. Optionally, a treatment plan may include detachment parameters, e.g., duration and/or intensity of detachment at a particular location.

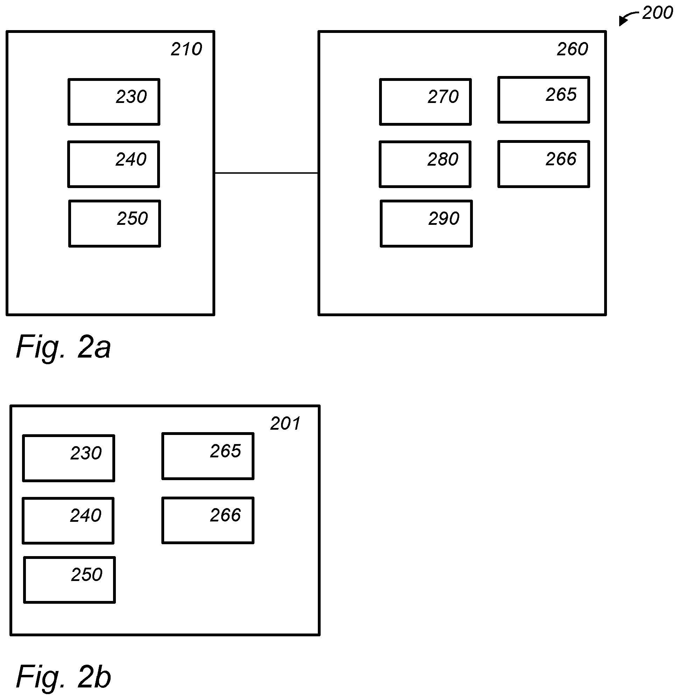

**Figure 2a** schematically shows an example of an embodiment of a treatment system 200. Treatment system 200 comprises a treatment planning device 210 and a treatment device 260. Treatment device 260 is configured for executing a tissue treatment at a defined location on a tissue slice. Multiple locations may be defined for a tissue slice. For example, treatment may comprise a detachment treatment. For example, detachment may be done chemically, e.g., using lysing, or mechanically, e.g., scraping.

In an embodiment, the tissue section is paraffined and/or formalin fixed. It is not necessary to restrict to FFPE tissue.

Treatment planning device 210 may comprise a processor system 230, a storage 240, and a communication interface 250. Treatment device 260 may comprise a processor system 270, a storage 280, and a communication interface 290. Treatment device 260 may further comprise treatment apparatus 265 and a camera 266. For example, the treatment apparatus 265 may comprise a mechanism to perform a treatment at a defined location, e.g., a lysing unit or the like. For example, camera 266 may be configured to image the tissue slice. In particular, camera 266 may be configured to image a first tissue slice having markings indicating one or more areas of interest, and a second tissue slice on which treatment is to be performed on the areas of interest indicated in the first tissue slice.

The first and second tissue slice could be the same slice, but typically, first and second tissue slice are different tissue slices, though typically from the same tissue, e.g., the slice may be neighboring slices, or even consecutive slices of a tissue.

The treatment apparatus 265 may be configured to perform the treatment operations, e.g., moving the treatment arm, and treatment at a location of the tissue section.

Storage 240 and/or 280 may comprise local storage, e.g., a local hard drive or electronic memory. Storage 240 and/or 280 may comprise non-local storage, e.g., cloud storage. In the latter case, storage 240 and/or 280 may comprise a storage interface to the non-local storage.

Treatment planning device 210 and/or treatment device 260 may communicate internally, with each other, with other systems, external storage, input devices, output devices, and/or one or more sensors over a computer network. The computer network may be an internet, an intranet, a LAN, a WLAN, etc. The computer network may be the Internet. The system comprises a connection interface which is arranged to communicate within the system or outside the system as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna.

For example, in an embodiment, treatment device 260 takes an image of a tissue slice. Treatment planning device 210 performs treatment planning using the image. For example, the image may indicate an area of interest in the tissue. The treatment planning aims to produce a set of geometric shapes covering the area of interest, possibly subject to various constraints, as discussed herein. A treatment plan may be generated, e.g., the plan may indicate the type of treatment which is to be applied at which location of the image.

In system 200, the communication interfaces 250 and 290 may be used to send or receive digital data. For example, treatment device 260 may send digital images representing tissue slices to treatment planning device 210. For example, treatment planning device 210 may send a treatment plan to treatment device 260.

The area of interest could be obtained in various ways. For example, a user, e.g., a pathologist, may indicate on the tissue an area of interest, e.g., using a marker pen. The markings may be detected using image processing algorithms, and an area of interest may thus be obtained. For example, a user, e.g., a pathologist, may indicate on an input device, e.g., a drawing tablet, the area of interest. This avoids the need to extract the area of interest with image processing. For example, an area of interest may be generated by a machine learned algorithm. For example, a neural network may be trained on a training set of tissue images and areas of interest, and learn to predict an area of interest. The machine learned algorithm may produce an area of interest, e.g., in the form of a binary mask indicating the area of interest, or a contour surrounding the area of interest or the like.

Whatever the source of the area of interest, there is still a need for generating a treatment plan, e.g., for detaching or otherwise treating the area of interest. Note that for a neural network conventional image segmentation algorithms may be used to extract of predict an area of interest, e.g., a convolutional neural network, e.g., a U-net, but those networks do not produce a treatment plan. Moreover, as treatment plans may be generated to support different constraints, training a neural network to do this is hard.

The execution of system 200, treatment planning device 210 and/or treatment device 260 may be implemented in a processor system, e.g., one or more processor circuits, e.g., microprocessors, examples of which are shown herein. The processor system may comprise one or more GPUs and/or CPUs. System 200, and/or devices 210 and 260 may comprise multiple processors, which may be distributed over different locations. For example, system 200 may use cloud computing.

System 200, treatment planning device 210 and/or treatment device 260 may comprise functional units that may be functional units of the processor system. For example, these may be used as a blueprint of a possible functional organization of the processor system. The processor circuit(s) are not shown separate from the units in some of the figures. For example, the functional units shown in figures 2a may be wholly or partially implemented in computer instructions that are stored at system 200, treatment planning device 210 and/or treatment device 260, e.g., in an electronic memory thereof, and are executable by a microprocessor thereof. In hybrid embodiments, functional units are implemented partially in hardware, e.g., as coprocessors, e.g., image processor for analyzing digital images including a defined location, and partially in software stored and executed on system 200.

**Figure 2b** schematically shows an example of an embodiment of a treatment system 201 in which treatment planning device and treatment device are integrated. For example, a processor system 230, a storage 240, and a communication interface 250 may be configured to perform the tasks needed for the treatment part of system 201 in addition to the treatment planning tasks.

In an embodiment, a treatment device, e.g., treatment device 260, combined device 201, is configured to receive a slide having a tissue section applied on the slide surface. For example, the slide may be a glass slide, or some other appropriate material. The treatment device is further configured to perform treatment at a defined location on the tissue section, e.g., using a motorized treatment tip and imaging the tissue section(s) using a camera. The defined location may be indicated in a treatment plan.

The images may show the area of interest, and typically also the surrounding tissue, or part thereof. The images may have a fixed perspective, e.g., obtained from a camera at a fixed location. The tissue slices may not be at a fixed position relative to the slides. The relative position of the slides to the camera may be easier fixed, but may not be entirely fixed either. Both positional variations can be resolved with image processing, e.g., registration algorithms.

For example, a treatment plan may comprise a plurality of locations, e.g., coordinates, and a corresponding plurality of parameters. For example, a parameter may be the size of the treatment tip, the intensity of the treatment, the duration of the treatment, and so on.

For example, a treatment plan may overlay the area of interest with one or more detachment shapes, corresponding to a treatment tip; in an embodiment, multiple detachment shapes are supported. A detachment shape may be circular, although this is not necessary.

For example, a packing algorithm may define multiple locations, which together detach, e.g., lyse, the tissue section, e.g., at the area of interest or a part thereof. For example, a circle packing algorithm may be used if the area at the defined location is circular. A packing algorithm improves the yield of the collected lysate. For example, a user may define an area for treatment that is larger than a single treatment area. The packing algorithm may select multiple locations in the area for treatment so that the corresponding treatment areas occupy the area for treatment. For example, the packing algorithm may perform multiple optimization iterations to improve a packing of treatment area in the area for treatment. Appropriate packing algorithms are defined herein.

The locations defined for the first image can be transferred to the second image. The transferred locations can then be used to steer the treatment arm and tip.

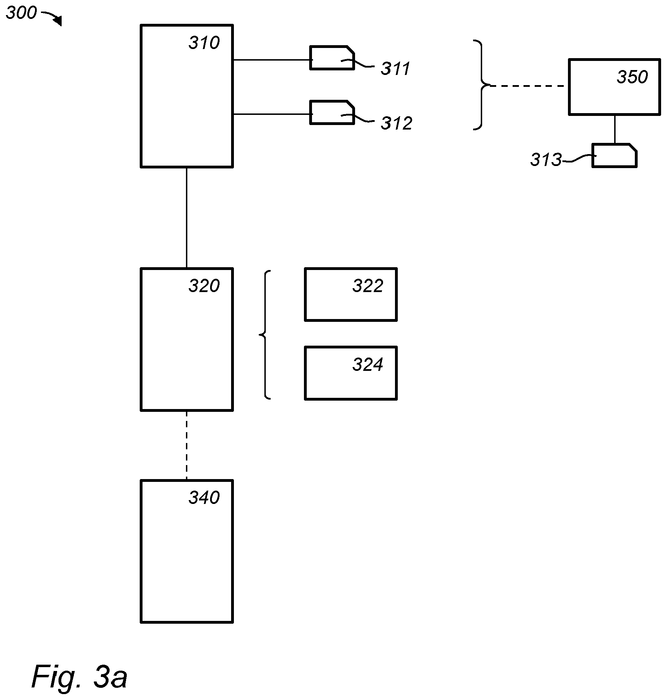

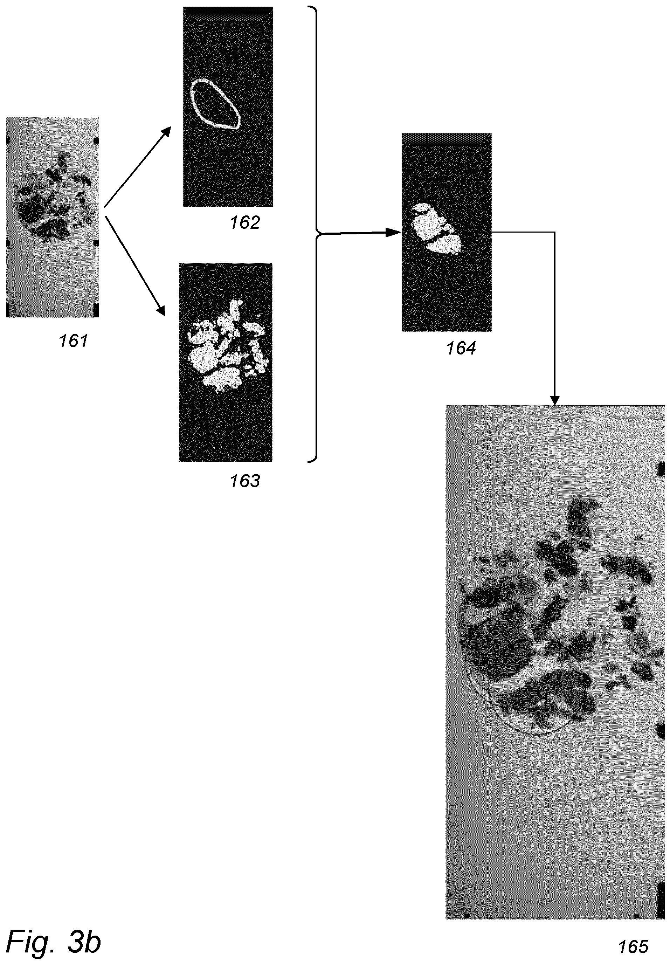

**Figure 3a** schematically shows an example of an embodiment of a treatment planning system 300. Treatment planning system 300 may be a stand-alone service, e.g., not coupled to a tissue treatment device. For example, system 300 may be implemented in a server, in the cloud, or the like, and configured to receive two or more images for alignment, and to return a transform between the images. System 300 may be implemented in a single device or distributed over multiple devices. System 300 may be combined, e.g., integrated, with a tissue treatment device. System 300 may be implemented in device 210 or 201.

System 300 may be configured to obtain an image 311 indicating an area of interest, for which planning is needed. For example, the area of interest(s) may be indicated by closed contours or curves in an image. System 300 may be configured to obtain an image 312 indicating the tissue section on which treatment may be performed, and which comprises the area of interest.

The image 311 and 312 may be obtained from an image filtering unit 350. Image filtering unit 350 may be part of treatment planning system 300, but need not be. Image filtering unit 350 may be configured to obtain an image 313 of a tissue section together with area of interest markings. The latter marking may be applied with a felt tip marker pen say. Image filtering unit 350 is configured to isolate from image 313 an image 311 with only the contours indicated by the user in image 313. Typically, image filtering unit 350 also isolates from the image 313 the image 312 with only the tissue parts. An advantageous implementation is to make contour image 311 and tissue image 312 masks. A mask contour image 311 is a binary image that indicates which pixel in the original image 313 correspond to a contour. A mask tissue image 312 is a binary image that indicates which pixel in the original image 313 correspond to a tissue. Typically, image 313 is a photo. Image 313 may also be supplied to system 300, especially if masks are used for images 311 and 312. Extracting of segmenting the area of interest or the tissue images from image 313 may advantageously be done with a neural network, e.g., a convolutional neural network, e.g., a U-net.

It may happen that the contours in image 313 that indicate the area of interest are not closed, but contain gaps. In that case, image processing can detect the endpoints of the partial contours and close the area of interest. Alternatively, a human operator could perform the closing of such partial contours. Closing contours may be done in treatment system 300 or in image filter 350.

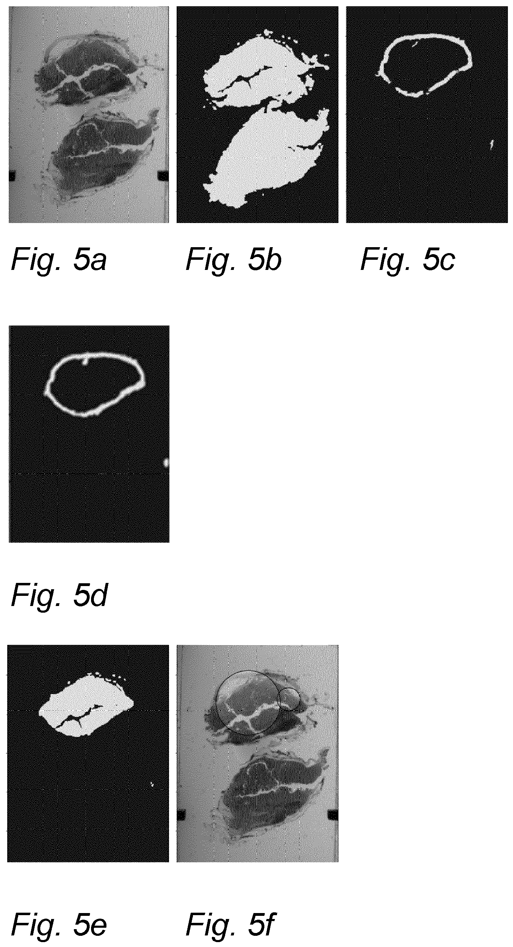

There are several ways to obtain images 311 and/or 312 from input image 313. For example, markings may be applied using a marker with a contrasting color. The image filtering unit 350 may then isolate those parts of the image that correspond to the contrasting color. For example, it turns out that green markings are easier to detect than black markings. The markings in the photos shown in grey scale, e.g., image 161 and figure 5a, are originally in green.

Another way to obtain images 311 and/or 312 which is to train a machine learned model to identify these parts of an image. For this approach a deep neural network was trained on a set of training images 313 and user isolated contours and tissue images. Surprisingly, much superior quality isolation is obtained than is possible with color-based isolation. In an embodiment, a neural network receives a color image 313 and produces a mask contour image 311. Producing a mask works well with a neural network, as only one output per pixel is needed. For example, the neural network image may be thresholded to obtain the mask image. A second neural network may be trained to produce a tissue mask image in the same manner. The first and second neural network can also be combined into a single network producing contour image and tissue image as output. The first and second neural network can also be partially combined with a common base, e.g., the initial layers, but a different head.

In an embodiment, a convolutional neural network (CNN), e.g., a Multi-Residual network (ResNet), is used for detecting the contours and/or for detecting the tissue. For example, a U-Net may be used. The neural network, e.g., the u-net, may be trained on a more general class of images, e.g., biomedical image segmentation, after which it may be finetuned on a training set to identify contours and/or tissue. An example U-net is the "U-Net: Convolutional Networks for Biomedical Image Segmentation", by O. Ronneberger and P. Fischer and T. Brox, included herein by reference.

Yet another way to obtain a contour mask, is to use different input modality. For example, a user may indicate the contours on a drawing tablet or the like. In this case, no isolation is needed, as the contours can be separated at creation. In this case, image filter 350 is not needed.

Treatment planning system 300 may comprise an image preprocessing part 310. Image preprocessing part 310 may preprocess the images that are received. For example, a received image may not yet be segmented into area of interest markings or contours and tissue. For example, the contours indicating an area of interest may not close up, so that endpoint may be detected, matched up, and a connecting segment drawn in. Further and/or other preprocessing is described herein.

Treatment planning system 300 may comprise a treatment planning unit 320 to perform the treatment planning. Treatment is typically detachment of the tissue, and more typically by lysing, but neither is necessary. Planning unit 320 may be configured to place detachment areas, e.g., geometric shapes corresponding to a detachment chamber that may be placed by a treatment device, on the tissue. The optimal allocation of detachment areas is a mathematically hard problem, but algorithms have been devised to make this problem tractable, while still producing very efficient solutions. For example, in an embodiment, a location for a detachment area is determined one at a time.

Planning unit 320 may comprise a distance determination and distance transform unit 322. For example, for each pixel that is not yet covered in the area of interest, a distance may be computed to the nearest edge of the area of interest. A gain and constraints determination unit 324 may compute for each point how large the gain would be if a detachment area were placed there. It may also compute if any particular constraints are violated. For example, a fluid based detachment chamber cannot be placed on the edge of the tissue slide, as the fluid would leak out of the detachment chamber.

Interestingly, one would expect that computing gain and constraints would be sufficient. Indeed, it is possible to make a working embodiment on that basis. For example, in each iteration an algorithm may place a detachment area on the location where the largest gain is to be had. However, it turned out that it is beneficial to give a strong preference toward placing the detachment area on the edge of the area of interest. This tends to enable a more efficient placement of later detachment areas. Accordingly, placement depends both on the distance to the edge, where a distance equal to the radius of the detachment area is preferred, while also aiming for large gain, and unviolated constraints.

Once a treatment plan is made, it can be forwarded to a treatment unit 340, e.g., a treatment device as shown in figure 1, 2a, and 2b.

Below several further optional refinements, details, and embodiments are illustrated.

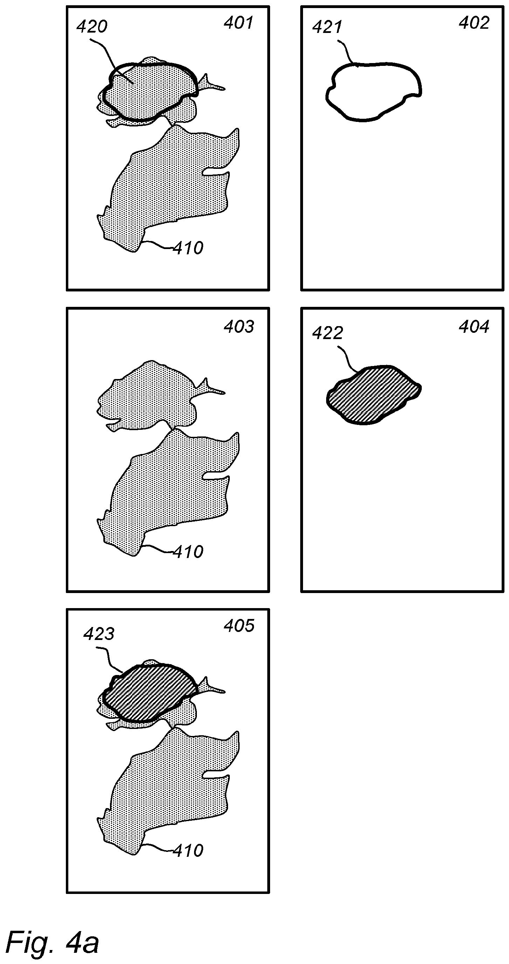

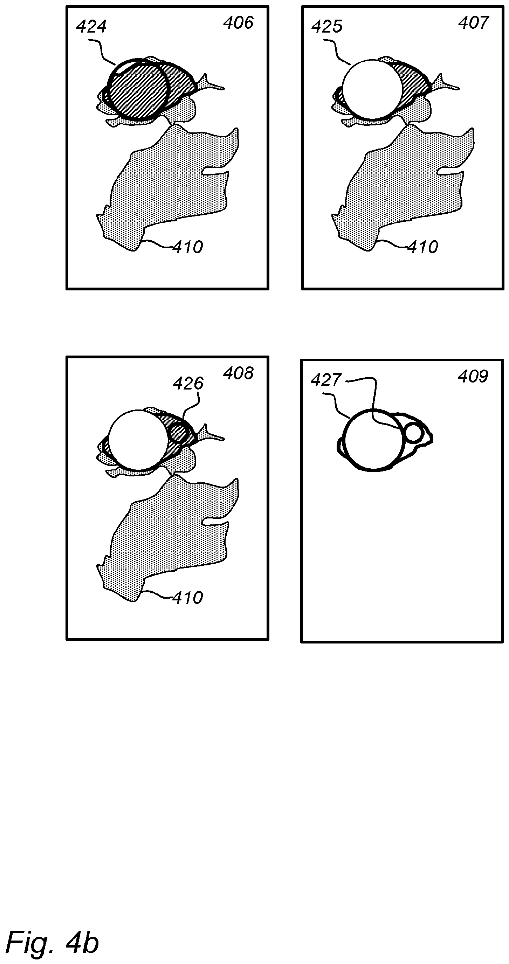

**Figures 4a and 4b** schematically show an example of an embodiment of treatment planning.

Treatment planning is useful to configure a treatment device to perform the tissue treatment. A typical treatment is to detach tissue by applying a lysing fluid in a detachment chamber placed at a defined location on the tissue; for example, detachment of an area of interest on a tissue section. However, other localized treatment is possible, e.g., localized application of a fluid interacting with the tissue, e.g., staining, chemical reactions, and the like. Local treatment is typically performed with a fluid, but this is not necessary, treatment could be heat treatment, UV treatment or the like.

In an embodiment, tissue treatment comprises detaching in a detachment area. This may comprise applying lysing chambers having the geometric shapes corresponding to detachment areas, dispensing a detaching liquid to the lysing chamber, allowing the detaching liquid to detach tissue, aspirating the detaching liquid with the detached tissue from the cavity, and forwarding the liquid for further processing. Further processing may comprise extracting DNA from the tissue. The detaching liquid may be dispensed and/or aspirated from a pipette tip arranged at a motorized pipettor arm, said arm being arranged to move the pipette tip to the cavity.

For example, a treatment device may comprise a motorized treatment tip, the motorized treatment tip being arranged for detaching multiple detachment areas having a geometric shape. Treatment may be planned for a treatment device configured for a single type of detachment chamber, e.g., one size and one shape. However, a treatment device may also support multiple types of detachment chamber. Typically, these are multiple sizes, though of the same shape, typically circular. However, a treatment device may also support multiple different shapes; For example, different shaped ovals. For example, different shaped polygons.

If a treatment device supports a shape that is not rotation symmetric, e.g., an oval then planning the treatment may comprise rotating the shape.

Various embodiments support treatment device of increasing complexity, with enabling increasingly complicated shapes, e.g., different shaped treatment chambers, e.g., detachment chambers. Even for a single sized, single shaped treatment device, a planning device is advantageous. Because of the large number of locations where a chamber may be placed, possibly allowing overlap, possibly allowing extending beyond the area of interest, and so, it was found that automated placement is both faster than human placement and provides treatment plans that better fulfill the desired properties, e.g., the goal of the treatment.

Planning and treatment does not need to be done with respect to the same tissue slice. For example, in an embodiment may comprise receiving a first tissue slice on the first tissue slice an area of interest being marked, and imaging the first tissue slice and obtaining the one or more images therefrom. The embodiment may further comprise receiving a second tissue slice, and detaching from the second tissue slice according to a treatment plan determined from the first tissue slice. Instead of receiving the first tissue slice only an image of the first tissue slice may be received.

In an embodiment of a planning device for a treatment device that supports at least two different geometric shapes, and/or at least two different sized shapes, multiple iterations may be performed for the possible treatment area shapes. For example, in a first set of iterations, locations are found for a first shape. Once the possibilities for the first shape are exhausted, a second set of iterations is performed for the second shape.

For the sake of simplicity, it will be assumed that the geometric shapes supported by the treatment device are circles, or discs. Furthermore, that the treatment devices support at least two sizes, say four sizes. Embodiments described herein may be adjusted for other types of treatment devices. For example, if a treatment device supports only one shape, then consideration, e.g., iterations, about other shapes may be eliminated from an embodiment to adjust to it to the simpler treatment device. Likewise, it will be assumed that tissue treatment is lysing with a lysing fluid in lysing chamber placed on the tissue at a defined location.

**Figure 4a, Image 401** schematically shows an image a tissue section 410. The image may have been taken with a camera from a tissue slide. On the image, a user, say a pathologist, has indicated an area of interest 420. It is the intention to detach the tissue in area 420. Note that the actual detachment may be performed from a further tissue section, e.g., a tissue section taken near the shown tissue section. This is useful, as the shown tissue section may be stained, while the tissue section used for detachment may be unstained.

**Figure 5a** schematically shows an example of an embodiment of an image showing tissue and markings. Figure 5a is a grayscale photo taken from an actual image section with markings.

From the image 401, various further images may be derived, e.g., a tissue image 403 showing the tissue 410 and a contour image 402 showing the area of interest markings indicating area 420. For example, a neural network trained for image segmentation may be trained to identify tissue section and to identify markings.

As shown in image 401 the area of interest is fully surrounded. It may happen that the user does not fully surround the area, but leave a gap somewhere. This may happen, e.g., if the user does not connect the start and finish of the curve. In that case, image processing may detect and match the endpoints and draw a connecting segment between them.

It is efficient to work on images in a computer. Even if the values are not naturally imageable values, e.g., scores, distances, and so on, then still one may refer to this 2D data as an image, although matrix or map are also used. One element of the data may then be referred to as a pixel. In this case a pixel may be understood as a possible placement for a treatment area. If the input images, e.g., is of higher resolution, it may be scaled down, or the number of pixels that are taken into account may be scaled down.

**Figure 4a, Image 402** shows a contour 421 indicating the area of interest, as indicated by the user. **Figure 5c** schematically shows an example of an embodiment of an image showing partial contours. In this example, the contour does not fully connect, e.g., through insufficient pressure during the marking. **Figure 5d** schematically shows an example of an embodiment of an image showing a completed contour. The partial contour segmented out from figure 5a is completed using image processing, to produce figure 5d.

**Figure 4a, Image 403** schematically shows an example of an embodiment of an image showing a tissue mask. **Figure 5b** schematically shows an example of an embodiment of an image showing a tissue mask.

The intersection 422 of tissue shown in image 403 and the area of interest of image 402 is shown in image 404. The contour shown in image 404 shows the tissue that is to be treated, e.g., detached. The tissue in image 403 and the area in image 404 may be represented as a mask, e.g., a binary image that indicates which pixels in image 401 belong to the tissue and/or area. **Figure 5e** schematically shows an example of an embodiment of an image showing an isolated area of interest; Figure 5e is the intersection of the tissue indicated in figure 5b and the area of interest indicated by the completed contour in figure 5d.

**Figure 4a, Image 405** shows the processed (intersected) area of interest 423 superimposed on the tissue image.

The images shown in figure 4a may be regarded as preprocessing in the sense that up to this point no decision about placement of detachment areas has been made. For example, an embodiment may comprise the following elements

1. A first tissue slice on a first slide is stained and used as a reference slide. A user, e.g., a pathologist, marks an area of interest (AOI) on the slide, e.g., using a permanent marker. Usually, the AOI corresponds to a tumor tissue in the biopsy.

2. A first image of the first slide is processed by two neural networks, producing a tissue mask and the contours of the area of interest. The output of the neural networks is typically a likelihood, e.g., between 0 and 1, indicating the likelihood that a pixel is tissue or contour. The neural network outputs are thresholded to obtain map of markings and of tissue. Preferably, the threshold for a marking is set higher than that for tissue, to avoid parts of the tissue being interpreted as a marking.

3. The contours in the markings are closed up using image processing, and then filled in and intersected with the tissue image to obtain an area of interest mask, also referred to as the area of interest map.

The intersection is to be covered with detachments shapes, typically different size circles.

**Figure 4b** schematically shows an example of an embodiment of treatment planning. **Figure 4b, Image 406** shows a first detachment area 424 placed on the area of interest in a first iteration.

**Figure 4b, Image 409** schematically shows an example of an embodiment of a finished treatment plan superimposed on the area of interest. **Figure 5f** schematically shows an example of an embodiment of an image showing a finished treatment plan superimposed on an image of a tissue section. Note that the treatment plans shown combine two different sized treatment areas. The treatment plan could also comprise a single treatment area or multiple treatment areas of the same size, or more than two treatment areas, and so on.

Computing an optimal placement of detachment areas according to some set of criteria is a complicated problem. There are hundreds if not thousands of pixels where a detachment area could be placed; moreover a plan may comprise multiple detachment areas, e.g., tens of them. Accordingly, iterating over all possible combined placements is not practical.

To avoid this combinatorial explications, in an embodiment, the possible detachment shapes are considered in turn, starting with the largest one. In each iteration, a position is search for the detachment area of that shape that will give good gain, satisfies conditions. Moreover, a position is sought that allowing future iterations to still find suitable positions. In particular, it was found that iteration should prefer placement near the edge of the area of interest. Although this could reduce gain, compared to a placement in the center, future iterations can be placed more advantageously thus making up for this.

An embodiment may work on a tissue mask and area of interest mask, or on only an area of interest mask. An embodiment may iterate the following set of elements one or more times. The set may be repeated for different detachment shapes, e.g., different sizes.

1. Compute a distance map. A distance maps indicates for each area of interest pixel the shortest distance to the edge of the area of interest

2. (Optional) Determine if the continuing with the present shape is likely to produce results.

3. Transform the distance map according to a distance transformation

4. Compute gain for each pixel, assuming a detachment area is placed there, and (optionally) verify other constraints.

5. Combine the distance transform and gain / constraints to produce a score for pixels, e.g., all pixels in the image, or all pixels in the area of interest.

6. Select that best scoring pixel as the location to place a detachment area.

7. Update the area of interest mask and/or tissue mask removing the placed detachment area

8. Continue with placing detachment areas, e.g., starting at element 1, until a stop criterion is reached, e.g., when a certain coverage of the area of interest is reached.

Possible implementations of these elements are discussed below.

**Compute a distance map.**

A distance maps indicates for each area of interest pixel the shortest distance to the edge of the area of interest. A distance map may be represented as a twodimensional array. In an embodiment, a distance to the nearest pixel in the tissue section outside the area of interest or in the background is computed for multiple pixels, e.g., the pixels in the area of interest, or the pixels in the tissue, or all pixels.

**(Optional) Determine if the continuing with the present shape is likely to produce results.**

From a maximal distance for the pixels in the area of interest, e.g., a distance to the nearest pixel in the tissue section outside the area of interest or background, it may be decided whether or not to perform the remainder of the iteration or to abort the remainder of the iteration. Note that the area of interest may shrink in subsequent iterations because of previously placed treatment areas.

If the area of interest is small compared to the treatment area, then this treatment area is too large. In that case, it is preferred to abort this set of iterations and proceed to the next smaller available size. If there is no next smaller size, the algorithm may continue, or alternatively, break off with a warning. Alternatively, say, if the area of interest's area is below a threshold, an alternative extraction method may be suggested, e.g., a manual extraction, e.g., a needle extraction.

For example, the maximal distance to an edge for the pixels in the area of interest may be computed, say, this value is *md.* A stop criterion for this iteration may be if *md · param < radius.* That is, if the largest distance to the area of interest edge times a factor is smaller than the current radius then skip this set of iterations, and continue with a smaller radius.

In a variation, a different value for *param* is taken for the first iteration, e.g., the very first placement with this size, then for the following iterations. Likewise, different parameters may be used for different pods. For the largest pod, a smaller parameter may be used.

For example, the *param* value may be 1.2 for the first nozzle placed, then 1.1 for the second. With a parameter of 1.2, the md is increased with 20%.

Instead of skipping iterations, one may compute the largest pod that fits in *md · param,* e.g., which has a radius smaller than the parameter times the max distance, and skip to that treatment area size.

Skipping part of the iterations has the advantage of producing treatment plans quicker.

**Distance transformation**

A distance transformation map the distances computed into a distance score by applying a distance transformation, e.g., a function. The distance transformation produces the best score if the distance of a pixel to the edge, is equal to the radius of the area of interest.

A distance transform may be a function *f(d)*, the values of which indicate how well-placed the pixel is positioned with respect to the border given the radius of the area of interest edge. For example, the function may have a value between 0 and 1, where the value 1 is best and 0 is worst; The ranges could be different.

For example, a distance transformation may be the function $f(d) = 1 - \dfrac{(R-d)^2}{R^2}$, wherein d is the distance to the edge, and R is the radius of the current detachment shape under consideration. An alternative function is

$$f(d) = 1 - \frac{abs(R-d)}{R}.$$

The former gives squared decay, the latter linear decay. Both will work, but squared decay gave better results. Other decay functions are possible for the distance transform. These two examples are symmetric with respect to the border; that is, crossing the border or stopping short of the border is penalized equally hard. This is not necessary, e.g., an asymmetric decay may be used. An asymmetric distance function is advantageous for some applications. For example, if detaching non-AoI tissue is undesirable, an asymmetric distance transform may penalized overlapping outside the AoI more than stopping short of the edge.

The above functions are symmetric with respect to the edge. This is not needed, for example, an alternate function may decay at a different rate for pixels inside the area of interest than for pixels outside the area of interest. Placement of detachment areas outside the area of interest is permissible, though will often result in worse gain.

Although the distance transform does not take gain explicitly into account, in practice a detachment area with a good score, e.g., 1 or close thereto, will typically have a large gain as well.

**Compute gain**

Optionally, one can estimate how effective the treatment would be at a particular location using a particular treatment shape. In particular, one can estimate how much the gain would be if detachment is done using a particular detachment shape. We will assume that gain is computed, but it can be changed to treatment effectiveness if desired.

It turns out that the distance transform on its own is already advantageous, at least on two fronts: it enables better placement of later treatment areas, without having the combinatorically consider all possible placement, moreover, a good score on the distance transform, e.g., placing a treatment at a location where the nearest point to the edge is close to the radius of the treatment area is also a proxy for a high gain.

However, especially at lower scores on the distance transform, an explicit computation of gain adds information. For example, if the nearest edge in one particular direction is quite close to a point, then the distance transform will give a bad score, but the gain may be quite good if in most other directions the edge is far away.

Another advantage to computing gain is to use it as a stopping criterium, for example, if gain is low, then the iterations for this shape may be terminated.

Gain can be effectively computed by convoluting the image with a kernel representing the shape of the treatment area. For example, if the treatment area is a disc, then the kernel may be a small image of a disc. If the treatment area has some other shape, then that maybe depicted in the kernel instead.

For example a gain map may be computed as a convolution matrix, e.g., a convolution with the kernel and the area of interest-or the remains of the area of interest since, previous detachments may remove parts thereof.

**Optionally compute constraints.**

In addition to gain other constraints may be evaluated for a pixel, where a treatment area may be placed. Such constraints may be incorporated in the gain, e.g., artificially lowering gain, even setting it to zero, if a constraint is violated. A separate constraints map could also be computed, e.g., with values of 1 for pixels that do not violate and values of 0 for pixels that do. Intermediate values are also possible, for example, between 0 and 1, for constraints that are not binary for preferential.

An example of a constraint is to forbid placement of treatment areas over the edge of the image. This would correspond to a treatment chamber over the edge of the tissue slide. For say a detachment chamber using a fluid, e.g., a detachment fluid, this will not work, as the fluid will leak away. A pixel that is closer to the edge of the image than the radius of the treatment area may be fully penalized, e.g., constraint value 0 or gain value set to 0.

Other constraints include allowing overlap between treatment areas. Overlap between treatment areas is typically not a problem, although it will lower the gain. But sometimes overlap is to be avoided, or is even forbidden. For any pixel the distance to a previous treatment area may be computed. If the distance is lower than the radius the placement may be forbidden, e.g., constraint value 0 or gain value set to 0, or discouraged, e.g., proportional to the amount of overlap, constraint value lower than 1 but above 0, say 0.5 or gain value reduced, say by 50%. The amount of reduction may be expressed as factor, say, a factor of 1 indicates no overlap is allowed; Lower values allow some overlap. A value of 0 has no explicit discouragement for overlap.

An important constraint includes allowing treatment outside the area of interest. For detachment allowing such treatment would mean that tissue not belonging to the area of interest gets mixed in with the intended tissue. Typically, this is not a problem if some unintended tissue gets mixed in. The algorithm will naturally discourage it, as treatment outside the area of interest does not contribute to gain, but in some situations a user, e.g., a pathologist, want to explicitly avoid such contamination with non-area of interest tissue.

For any pixel the overlap with non-area of interest may be computed, e.g., as an image intersection of the tissue mask minus the area of interest, and a kernel representing the treatment area. For example, this map may be computed as a convolution as well. As a proxy, the distance to the area of interest edge may be used, as it is already computed anyway as part of the distance transform. The amount of non-tissue maybe translated to a constraint score, for example if higher than a threshold, or higher than 0, the placement may be forbidden, e.g., constraint value 0 or gain value set to 0. Instead, placement may be discouraged, e.g., proportional to the amount of non-AoI tissue, e.g., by setting a constraint value lower than 1 but above 0, say 0.5 or gain value reduced, say by 50%.

The amount of reduction may be expressed as factor, say a factor of 1 indicates no overlap is allowed, Lower values allow some overlap. A value of 0 has no explicit discouragement for overlap.

Combine computed information into a score for pixels

An embodiment may compute one or more values for all or part of the pixels in the image range, e.g., the tissue or AoI image range. These values may include: a distance transform, a gain, an overlap constraint, a non-AoI constraint and so on. In an embodiment, two values are used, the distance transform value and a gain value. The gain value is modified down if a constraint is violated. Not all constraints are important for all applications.

These values are combined into a single score. This could be multivariate combining function, e.g., a convex multivariate combining function. In practice, a good choice for combining these scores is to multiply them.

Interestingly, for many pixels this combination will depend heavily on the distance transform-many pixels in the interior of the area of interest may have maximum gain and pass all constraints. In that case, the distance transformed values will be the major factor. Still the combined score has advantages, such as the ability to penalize placement that are placed beyond the edge of the image, or violate other constraints, e.g., non-AoI tissue, overlapping treatment areas, etc.

**Select that best scoring pixel**

When the scores for the relevant pixels are known, then the best scoring one may be selected. If there are ties, or near ties, e.g., within a threshold of each other, the pixels with the fewest non-AoI tissue overlap may be selected.

For example, an embodiment may be as follows:
Function: Creating_ScoreMap

1) Initialize **mapping:** Create a Zero matrix in the size of the image. (mapping matrix)
2) For each non-AoI tissue pixel, the mapping matrix receives a -1 at the same position. (This is to penalize the values for tissue)
3) For each AoI pixel the mapping gets a value of 1 (This makes it more profitable here).
4) The edge of the image is given a value of -100 (the penalty here is so high that a pod can never be placed over the edge)

5) The convolution matrix is computed as the mapping matrix convoluted with a filled circle with a radius of the treatment chamber (that is a kernel with value = 1 for pixels in the treatment area and zero elsewhere)

6) The score map is computed as: score = conv*sqdist

7) Pod position is placed, where score map is maximum

sqdist refers to distance of the pixel to the edge of the AoI to which a distance transform has been applied.

**Figure 4b, Image 406** shows a first detachment area 424 placed on the area of interest in a first iteration. Note that area 424 has some overlap outside the area of interest, but this part is chosen at a location where there happens to be no tissue. At the same time a location is found, that leaves room for future detachment areas.

**Update the area of interest mask and/or tissue mask removing the placed detachment area**

Once a treatment area is placed, e.g., as in image 406, the image may be updated to reflect that this part of the tissue is not treated. This may be done by removing the area from the tissue mask as well as from the area of interest mask.

For example, a single image may be used, in which a pixel value indicates background, non-AoI tissue, or AoI tissue.

Some variants are possible, for example, the treated area may be replaced with a value indicating background or with a value indicating tissue but not AoI. This has an impact on how future placements will interact with previous placements.

In a variant, a pixel may be given a value indicating AoI but already treated. When computing overlap with non-AoI such area could be weighted less negatively, or overlap in such area may not be discouraged at all beyond the reduced gain.

Figure 4b, image 407 shows an updated image, in which treated area 425 is replaced with background, non-tissue value. The algorithm will typically treat this area neutrally, not avoiding overlapping it, but will not seeking to cover it either.

**Continue with placing detachment areas**

At this point a next treatment area may be selected. For example, the algorithm for placing the first treatment area may be repeated for a next treatment area. At some point, the algorithm may stop trying to place a treatment shape, and move on to the next supported treatment shape, or stop altogether if none are available.

In an embodiment, iterations are performed for geometric shapes from largest radius to smallest radius. When the treatment areas are circles, the radius give full information. However, for other shapes similar computations can be performed.

For example, for an elliptic shape, the algorithm may be adapted with a rotational iteration, using the smaller radius which would need to be increased if the shape covers a tissue area outside of the AoI. Other shapes like rectangles could be handled similarly.

For example, an iteration may be repeated for the same shape but rotated over various degrees, e.g., from 0 - 360, with intervals of, 30 degrees, 10, degrees, 1 degree, or the like. The best placement over all rotations is then chosen. For example, a shape may be tried rotated over 0, 30, 60, ..., 330 degrees. For each rotation, a score is computed, and the maximum score over the considered pixels and rotation is selected.

An approach that may be used for non-circular shapes is to use the same distance transformation as for circular shapes. The radius used for that distance transform may be the radius of a circle approximating the geometric shape. For example, the approximating circle may be the smallest circle enclosing the non-circular shape, e.g., the circumradius. For example, the approximating circle may be the largest innercircle of the non-circular shape, e.g., the inradius. The radius may be an average of the circumradius and inradius.

Using an approximating circle works since the distance transformed value is not the only criterion for placing of a (non-circular) treatment area. Even when approximated with a different, e.g., larger, enclosing circle, the distance transformed value (sqdist) of still provides valuable information about good locations to place a Pod.

In the example of image 407, the larger treatment area did not have a suitable placement anymore, so a smaller treatment area was selected. This one was then placed at 426 in image 408. The final treatment plan 427 is shown in image 409.

Various stopping criterions are possible

1. percentage of the area to be extracted (AOI area covered)
If the amount of covered AoI tissue is high enough, no additional treatment areas are needed
2. Extraction of tissue outside the AOI (non-AOI area)
If the amount of covered non-AoI tissue is too high, e.g., above a threshold, the algorithm may terminate unsuccessfully, and report an error.
3. A maximum number of extraction cycles has been used.
4. An expected processing time for the treatment is reached.

For example, a user may choose if he prefers speed of detachment, completeness of detachment, or low non-AOI extraction.

For example, in an embodiment various profiles may be defined, with targets for AoI covered, maximum number of treatment areas, maximum coverage of non-

AoI and/or, maximum treatment time.

Gain may be used as a criterium for progressing to a next shape or for terminating all further iterations. There is an interesting variant that may be used in an embodiment. Before placing a treatment area, a minimum gain level may be defined, e.g., specific for that size of the treatment area. If placing a treatment area does not reach the gain threshold, then it is not placed. A treatment device may be able to reuse a detachment chamber multiple times however, e.g., a detachment chamber may be used four times. In an embodiment, if a detachment chamber is used for the first time, a high gain threshold may be used, than if the detachment chamber is reused. That is, a large gain is needed, otherwise the algorithm may advance to a smaller treatment shape. If a detachment chamber is used for a further time, a lower gain threshold may be used. Since no new resources are needed for the additional treatment, a lower threshold may be used. For example, in an embodiment, the first placement may need a gain of 75%, e.g., 75% of the treatment area should correspond to AoI tissue, but later placement, e.g., second to fourth use, require only a gain of 25%. If there is no smaller treatment shape available, the gain threshold may be lowered.

Below an example embodiment is presented:

1. An image is created with pixels values for tissue = 0; background = 150; aoi = 255
2. The AoI part is to be covered with different sized circles; with radii from large to small according to a given sequence
3. For each radius from large to small do the following

    a. Compute a distance map.

        i. A distance map computes for each pixel, the smallest distance to a tissue pixel
        ii. Find the maximum value, that lies in the AOI. This is the point in the AOI that is furthest removed from the edge of the AOI.
        iii. Then multiply it with a parameter value. Then check that the largest pod fits therein (has a radius smaller than the parameter times the max distance)

        Note different parameters may be used for different pods. For the first and largest pod, a smaller parameter may be used.
        At this stage, the pod is not actually placed. It is only checked if the pod could fit at all

    b. After this check, try to fit copies, preferring edge placement

        1. Compute a distance transform (bw is distance to tissue or background), e.g., sqdist = 1-(R-bw)**2/R**2 # squared decay, and obtain a score
        2. Pick a best score, place a pod

            a. A placed pod is filled in with background color, e.g., color=150. So will have impact on distance map.

        3. Continue placing this type of pod while improvements are obtained.
        4. Otherwise go to the next smallest pod

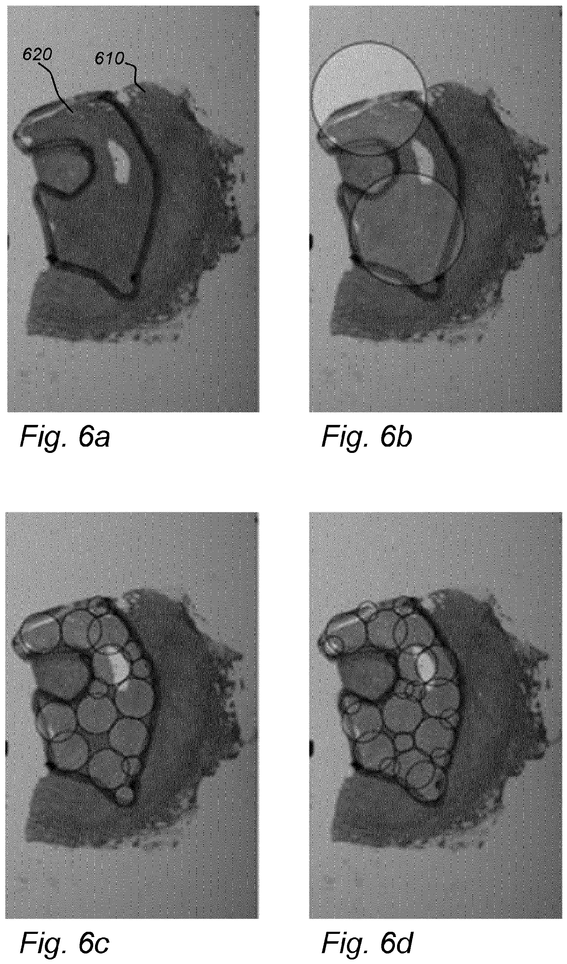

**Figure 6a** schematically shows an example of an embodiment of an image showing tissue and markings. Figure 6a was obtained as a photo of a tissue section, the markings are applied by a user. Figure 6a shows an area of interest 620 and non-AoI tissue 610.

**Figure 6b-6c** schematically shows an example of an embodiment of an image showing a treatment plan superimposed on an image of tissue. The treatment plans shown in figures 6b-6c were computed with an embodiment, but using different parameters.

Figure 6b was computed with a fast profile. The profile allows high overlap with non-AoI tissue and has a low target for AoI coverage. The treatment plan shown has an AOI Area Cov.: 77.1%, non-AOI Area Cov.: 10.9%. This treatment plan uses 2 extraction pods, and has a short extraction time. Note that this treatment plan uses large detachment areas that significantly overlap with non-AoI Tissue. The non-AoI coverage percentage is expressed as a percentage of the AoI area.

Figure 6c was computed with a medium profile. The profiles allow much less overlap with non-AoI tissue and has a higher target for AoI coverage. The treatment plan shown has an AOI Area Cov.: 82.9%, a non-AOI Area Cov.: 0.3%. Estimated extraction time is significantly longer than the treatment plan of figure 6b. This treatment plan uses 7 extraction pods. Note that the number of placements is higher, as some pods are reused. Note that much more detachment areas are used than in figure 6b, which are also smaller.

Figure 6d was computed with an accurate profile. Like the medium profile fewer overlap with non-AoI tissue is allowed then figure 6b. The profile has the highest target for AoI coverage. The treatment plan shown has an AOI Area Cov.: 91.7%, non-AOI Area Cov.: 1.1 %. Estimated extraction time is significantly longer than the treatment plan of figure 6c. This treatment plan uses 9 extraction pods.

Which profile to use depends on the requirements of the treatment, e.g., detachment. For example, a small area of interest may use a high AoI coverage target and low allowance for non-AoI, since this combination ensures that the DNA of interest will both be sufficient in quantity and not be contaminated too much. However, for a larger AoI, a lower coverage may be sufficient.

In an embodiment, AoI coverage target is ex-

pressed in area, e.g., square millimeter, instead of in a percentage.

The treatment plans shown in figures 6b-6d are made with the constraint that some pods may be used more than once. The number of of allowed uses, may differ depending on the pod, e.g., on its size. In this example, the biggest pod does not allow reuse. Accordingly, figure 6b shows both 2 placements and uses 2 pods. The smallest nozzle is allowed to be reused 4 times. The medium sized pod can be reused 2 times. Depending on pod size or needs of the treatment other limits on the number of times a pod can be reused may be used in the planning.

The extraction time depends on the number of placement, but also on the reagents used, and treatment at the tissue. For example, a reagent may be left to react longer or shorter with the tissue depending on the application.

Other parameters than can be taken into account when planning a treatment may include one or more or all of the following:

- Tissue type. For example, breast tissue or liver tissue have different demands when extracting.
- Number of unstained slides mounted into the device. If planning is done on a stained device, but extraction from unstained devices, then the amount of extraction desired is higher if the number of unstained slides is lower. For example, the AoI covered parameter may decrease as the number of unstained slides decreases, e.g., according to a function or table.
- Downstream process needs. For example, how much DNA or RNA is needed, e.g., in ng. For example, a targeted amplicon based NGS panels versus ligation based NGS

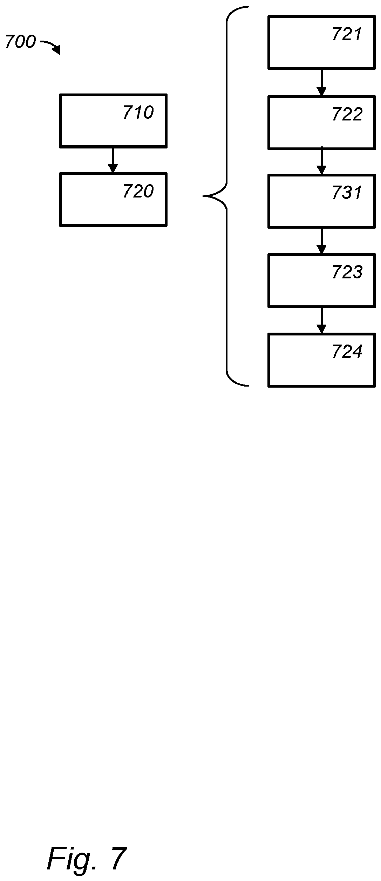

**Figure 7** schematically shows an example of an embodiment of a treatment planning method 700, in particular for planning detachment of an area of interest on a tissue section with a motorized treatment tip. The motorized treatment tip is arranged for detaching multiple detachment areas having a geometric shape. Method 700 comprises

- obtaining (710) one or more images representing a tissue section, an area of interest which is part of the tissue section, and background, and
- iteratively (720) selecting a position for a detachment area.

For example, the iteratively selecting may comprise

- computing (721) for each pixel in the area of interest a distance to the nearest pixel in the tissue section outside the area of interest or in the background,
- applying (722) a distance transform to the distance, the distance transform having a preferred value for a radius of the geometric shape and having less preferred values for values higher and/or lower than the radius,
- computing (731) an expected detachment gain from the selected detachment area,
- selecting (723) a position for a detachment area from the result of the distance transform and the expected detachment gain,
- recording (724) the selected position for a detachment area, and removing from the area of interest a geometric shape corresponding to a detachment area at the selected position.

Computing and/or using detachment gain is optional.

Many different ways of executing the method are possible, as will be apparent to a person skilled in the art. For example, the order of the steps can be performed in the shown order, but the order of the steps can be varied, or some steps may be executed in parallel. Moreover, in between steps other method steps may be inserted. The inserted steps may represent refinements of the method such as described herein or may be unrelated to the method. For example, some steps may be executed, at least partially, in parallel. Moreover, a given step may not have finished completely before a next step is started.

Embodiments of the method may be executed using software, which comprises instructions for causing a processor system to perform method 700. Software may only include those steps taken by a particular subentity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a fieldprogrammable gate array (FPGA), to perform the method.

Below a non-limiting list of examples is included, exemplifying the technology disclosed herein.

Example 1. A method (700) for planning detachment of an area of interest on a tissue section with a motorized treatment tip, the motorized treatment tip being arranged for detaching multiple detachment areas having a geometric shape, the method comprising

- obtaining (710) one or more images representing a tissue section, an area of interest which is part of the tissue section, and background,
- iteratively (720) perform

  - computing (721) for each pixel in the area of interest a distance to the nearest pixel in the tissue section outside the area of interest or in the background,

- applying (722) a distance transform to the distance, the distance transform having a preferred value for a radius of the geometric shape and having less preferred values for values higher and/or lower than the radius,
- selecting (723) a position for a detachment area from the result of the distance transform,
- recording (724) the selected position for a detachment area, and removing from the area of interest a geometric shape corresponding to a detachment area at the selected position.

Example 2. A method as in Example 1, wherein removing from the area of interest a geometric shape corresponding to a detachment area at the selected position comprises amending the images to marking said geometric shape as tissue section or as background.

Example 3. A method as in any one of the preceding examples, wherein the distance transform comprise a quadratic term $(R - d)^2$, or an absolute linear term $abs(R - d)$, e.g., $1 - (R - d)^2/R^2$, wherein $R$ represents the radius and d represent the distance. Example 4. A method as in any one of the preceding examples, comprising computing an expected detachment gain for detachment areas associated with a pixel, wherein selecting a position for a detachment area depends on the expected detachment gain. Example 5. A method as in Example 4, wherein the iteration is continued until the incremental gains are below a threshold.

Example 6. A method as in any one of the preceding examples, wherein the motorized treatment tip, is arranged for detaching areas having multiple different geometric shapes, the iteration being performed for at least two different geometric shapes.

Example 7. A method as in Example 6, wherein the iterations are performed for geometric shapes from largest radius to smallest radius.

Example 8. A method as in any one of the preceding examples, wherein the geometric shapes are circles.

Example 8.1. A method as in any one of Example 6-8, wherein if expected detachment gain is below a threshold, placement selecting for a next geometric shape is performed Example 8.2. A method as in any one of Example 6-8.1, wherein a placement for a geometric shapes is searched and the expected detachment gain is computed, if the expected detachment gain is below a threshold, placement for a next geometric shape is searched instead, wherein the threshold depends on whether the corresponding detachment chamber in the treatment plan is used for the first time or for a subsequent time.

Example 8.3. A method as in any one of the preceding examples, wherein a detachment chamber is reusable for a defined number of detachments, which may depend on the nozzle type, the method comprising maintaining the number of remaining reuses, and penalizing a placement of a detachment area corresponding to a first use of a detachment chamber, and/or incentivize a placement of a detachment area corresponding to a re-using of a detachment chamber.

Example 9. A method as in any one of the preceding examples, comprising computing a maximal distance for the pixels in the area of interest a distance to the nearest pixel in the tissue section outside the area of interest or background, and deciding from the maximal distance to perform the remainder of the iteration or to abort the remainder of the iteration.

Example 10. A method as in any one of the preceding examples, comprising computing for each pixel a constraints score indicating a suitability for receiving a detachment area, a final score being computed as a product of the constraints score and the transformed distance.

Example 11. A method as in Example 10, wherein the constraints score, penalizes a detachment area outside the area of interest and/or a detachment area outside a tissue slide section of the image.

Example 12. A method as in any one of the preceding examples, wherein detaching in a detachment area comprises applying lysing chambers having the geometric shapes corresponding to detachment areas, dispensing a detaching liquid to the lysing chamber, allowing the detaching liquid to detach tissue, aspirating the detaching liquid with the detached tissue from the cavity, and forwarding the liquid for further processing. Example 12.1. A method as in Example 12, wherein the detaching liquid is dispensed and/or aspirated from a pipette tip arranged at a motorized pipettor arm, said arm being arranged to move the pipette tip to the cavity

Example 12.2. A method as in Example 12 or 12.1 for tissue treatment, the method comprising

- receiving a first tissue slice and a second tissue slice, on the first tissue slice an area of interest being marked,
- imaging the first tissue slice and obtaining the one or more images therefrom,
- the detaching being performed on the second tissue slice as planned.

Example 12.3 A method as in any one of the preceding examples, wherein

- the area of interest is marked by a user, e.g., a pathologist,
- the area of interest is determined by a machine learnable model.

Example 12.4. A method as in Example 12, 12.1 or 12.3, the method comprising

- receiving a first image representing tissue and a second image with digital and/or analog markings, e.g., AoI annotation, or
- receiving an image representing tissue and digital and/or analog markings, e.g., AoI annotation,

- determining a treatment plan from the image, or from the first and second image.

Example 13. A system for planning detachment of an area of interest on a tissue section with a motorized treatment tip, the motorized treatment tip being arranged for detaching multiple detachment areas having a geometric shape, the system comprising: one or more processors; and one or more storage devices storing instructions that, when executed by the one or more processors, cause the one or more processors to perform operations for

- obtaining one or more images representing a tissue section, an area of interest which is part of the tissue section, and background,
- iteratively perform

  - computing for each pixel in the area of interest a distance to the nearest pixel in the tissue section outside the area of interest or in the background,
  - apply a distance transform to the distance, the distance transform having a preferred value for a radius of the geometric shape and having less preferred values for values higher and/or lower than the radius,
  - select a position for a detachment area from the result of the distance transform,

- recording the selected position for a detachment area, and removing from the area of interest a geometric shape corresponding to a detachment area at the selected position.

Example 14. A system as in Example 13 comprising a motorized treatment tip arranged to chemically or physically treat a tissue slice, the operations including

- imaging the first tissue slice and obtaining the one or more images therefrom,
- performing detaching on the second tissue slice as planned with the motorized treatment tip.

Example 15 A transitory or non-transitory computer readable medium (1000) comprising data (1020) representing instructions, which when executed by a processor system, cause the processor system to perform the method according to any one of examples 1-11.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

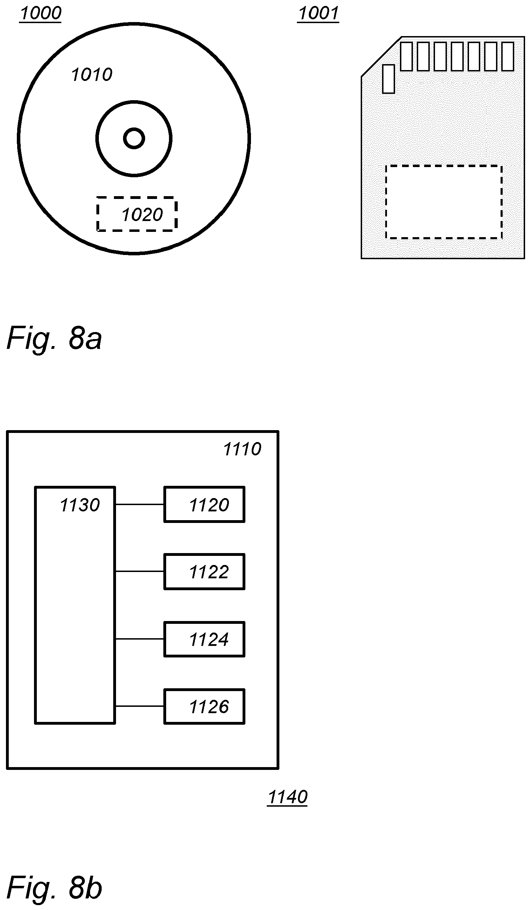

**Figure 8a** shows a computer readable medium 1000 having a writable part 1010, and a computer readable medium 1001 also having a writable part. Computer readable medium 1000 is shown in the form of an optically readable medium. Computer readable medium 1001 is shown in the form of an electronic memory, in this case a memory card. Computer readable medium 1000 and 1001 may store data 1020 wherein the data may indicate instructions, which when executed by a processor system, cause a processor system to perform an embodiment of a method of planning, e.g., determining a treatment plan, and/or executing a treatment plan, according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said method of planning, determining a treatment plan, and/or executing a treatment plan.

**Figure 8b** shows in a schematic representation of a processor system 1140 according to an embodiment of a system for planning, determining a treatment plan, and/or executing a treatment plan. The processor system comprises one or more integrated circuits 1110. The architecture of the one or more integrated circuits 1110 is schematically shown in Figure 8b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors, or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the system for closing a contour, determining a treatment plan, and/or executing a treatment plan device may comprise a processor circuit and a memory circuit, the processor being arranged to execute software stored in the memory circuit. For example, the processor circuit may be an Intel Core i7 processor, ARM Cortex-R8, etc. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of" when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

**Claims**

1. A method (700) for planning treatment of an area of interest on a tissue section with a motorized treatment tip, the motorized treatment tip being arranged for treating multiple treatment areas having a geometric shape, the method comprising

   - obtaining (710) one or more images representing a tissue section, an area of interest which is part of the tissue section, and background,
   - iteratively (720) perform

      - computing (721) for each pixel in the area of interest a distance to the nearest pixel in the tissue section outside the area of interest or in the background,
      - applying (722) a distance transform to the distance, the distance transform having a preferred value for a radius of the geometric shape and having less preferred values for values higher and/or lower than the radius,
      - selecting (723) a position for a treatment area from the result of the distance transform,
      - recording (724) the selected position for a treatment area, and removing from the area of interest a geometric shape corresponding to a treatment area at the selected position.

2. A method as in Claim 1, wherein removing from the area of interest a geometric shape corresponding to a treatment area at the selected position comprises amending the images to marking said geometric shape as non-area of interest, e.g., as tissue section or as background.

3. A method as in any one of the preceding claims, wherein the distance transform comprise a quadratic term $(R - d)^2$, or an absolute linear term $abs(R - d)$, e.g., $1 - (R - d)^2/R^2$, wherein $R$ represents the radius and d represent the distance.

4. A method as in any one of the preceding claims, comprising computing an expected treatment gain for the pixels, wherein selecting a position for a treatment area depends on the expected treatment gain.

5. A method as in Claim 4, wherein the iteration is continued until the incremental gains are below a threshold.

6. A method as in any one of the preceding claims, wherein the motorized treatment tip, is arranged for treating areas having multiple different geometric shapes, the iteration being performed for at least two different geometric shapes.

7. A method as in Claim 6, wherein the iterations are performed for geometric shapes from largest radius to smallest radius.

8. A method as in any one of the preceding claims, wherein the geometric shapes are circles.

9. A method as in any one of the preceding claims, comprising computing a maximal distance for the pixels in the area of interest a distance to the nearest pixel in the tissue section outside the area of interest or background, and deciding from the maximal distance to perform the remainder of the iteration or to abort the remainder of the iteration.

10. A method as in any one of the preceding claims, comprising computing for each pixel a constraints score indicating a suitability for receiving a treatment area, a final score being computed as a product of the constraints score and the transformed distance.

11. A method as in Claim 10, wherein the constraints score, penalizes a treatment area outside the area of interest and/or a treatment area outside a tissue slide section of the image.

12. A method as in any one of the preceding claims, wherein treating in a treatment area comprises detaching of tissue in a detachment area.

13. A method as in Claim 12, comprising applying lysing chambers having the geometric shapes corresponding to detachment areas, dispensing a detaching liquid to the lysing chamber, allowing the detaching liquid to detach tissue, aspirating the detaching liquid with the detached tissue from the cavity, and forwarding the liquid for further processing.

14. A system for planning treatment of an area of interest on a tissue section with a motorized treatment tip, the motorized treatment tip being arranged for treating multiple treatment areas having a geometric shape, the system comprising: one or more processors; and one or more storage devices storing instructions that, when executed by the one or more processors, cause the one or more processors to perform operations for

   - obtaining one or more images representing a tissue section, an area of interest which is part of the tissue section, and background,
   - iteratively perform

      - computing for each pixel in the area of interest a distance to the nearest pixel in the tissue section outside the area of interest or in the background,
      - apply a distance transform to the distance, the distance transform having a preferred value for a radius of the geometric shape and having less preferred values for values higher and/or lower than the radius,
      - select a position for a treatment area from the result of the distance transform,

   - recording the selected position for a treatment area, and removing from the area of interest a geometric shape corresponding to a treatment area at the selected position.

15. A system as in Claim 14 comprising the motorized treatment tip, the operations including

   - imaging the first tissue slice and obtaining the one or more images therefrom,
   - performing treatment on the second tissue slice as planned with the motorized treatment tip.

16. A transitory or non-transitory computer readable medium (1000) comprising data (1020) representing instructions, which when executed by a processor system, cause the processor system to perform the method according to any one of claims 1-13.

Fig. 1

*200*

| 210 | | 260 |
|---|---|---|
| 230 | 270 | 265 |
| 240 | 280 | 266 |
| 250 | 290 | |

## Fig. 2a

| 201 | |
|---|---|
| 230 | 265 |
| 240 | 266 |
| 250 | |

## Fig. 2b

*Fig. 3a*

*161*

*162*

*163*

*164*

*165*

*Fig. 3b*

*Fig. 4a*

Fig. 4b

Fig. 5a

Fig. 5b

Fig. 5c

Fig. 5d

Fig. 5e

Fig. 5f

Fig. 6a

Fig. 6b

Fig. 6c

Fig. 6d

*700*

*Fig. 7*

1000

1001

1010

1020

Fig. 8a

1110

1130

1120

1122

1124

1126

1140

Fig. 8b

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/085443 A1 (KALLIONIEMI OLLI P [FI] ET AL) 6 May 2004 (2004-05-06)<br>* paragraphs [0155], [0196], [0291], [0324], [0327], [0328], [0371], [0380]; claims 1,5,6,8,12,13,21,44,58,59,64; figures 10,15,16,17 *<br>----- | 1-16 | INV.<br>G01N1/31<br>C12N1/06<br>C12N15/10<br><br>ADD.<br>G01N1/08<br>G01N1/34<br>G01N35/10 |
| A | EP 2 580 348 B1 (QIAGEN GMBH [DE]) 25 April 2018 (2018-04-25)<br>* paragraph [0044] *<br>----- | 13 | |

**TECHNICAL FIELDS SEARCHED (IPC)**

G01N

The present search report has been drawn up for all claims

| Place of search | Date of completion of the search | Examiner |
|---|---|---|
| The Hague | 15 May 2023 | Geromel Prette, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 3113

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2023

| Patent document cited in search report | | | Publication date | Patent family member(s) | | | Publication date |
|---|---|---|---|---|---|---|---|
| US 2004085443 | A1 | | 06-05-2004 | NONE | | | |
| EP 2580348 | B1 | | 25-04-2018 | EP | 2580348 | A1 | 17-04-2013 |
| | | | | JP | 2013531987 | A | 15-08-2013 |
| | | | | JP | 2017029171 | A | 09-02-2017 |
| | | | | JP | 2018186836 | A | 29-11-2018 |
| | | | | JP | 2020048579 | A | 02-04-2020 |
| | | | | JP | 2022009735 | A | 14-01-2022 |
| | | | | US | 2013095473 | A1 | 18-04-2013 |
| | | | | WO | 2011157678 | A1 | 22-12-2011 |

EPO FORM P0459

For more details about this annex : see Official Journal of the European Patent Office, No. 12/82

**EP 4 386 352 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020131070 A
- WO 2020131072 A
- WO 2020132394 A
- WO 2020254250 A1